# EUROPEAN PATENT APPLICATION

(11) **EP 4 067 882 A1**
(43) Date of publication of application: **05.10.2022**
(21) Application number: 20893432.3
(22) Date of filing: 26.05.2020
(51) Int. Cl.: G01N 21/84

(54) **OPTICAL UNIT**

(30) Priority: 27.11.2019 JP 2019213963
(71) Applicant: Synqroa Co., Ltd., Toshima-ku, Tokyo 171-0042 (JP)
(72) Inventor: KOYAMA, Mitsuhiro, Tokyo 171-0042 (JP); AYABE, Kaori, Tokyo 171-0042 (JP)
(74) Representative: Schäfer, Matthias W.
(86) International application number: PCT/JP2020/020674
(87) International publication number: WO 2021/106244

(57) **Abstract**

An optical unit 1 of the present invention includes a light source 5 configured to irradiate an object with light, a first polarizing element 11 configured to convert emitted light emitted from the light source 5 to linearly polarized light, a second polarizing element 16 configured to cause reflection light from the object to transmit through the second polarizing element 16, a polarization axis of the second polarizing element 16 being 90° orthogonal to a polarization axis of the first polarizing element 11, a first wavelength plate 12 disposed to overlap with the first polarizing element 11 and configured to convert the emitted light from the light source to circularly polarized light or elliptically polarized light, and a second wavelength plate 17 disposed to overlap with the second polarizing element 16, a phase difference of the second wavelength plate 17 being equal to a phase difference of the first wavelength plate 12. The first wavelength plate 12 is revolvable with respect to the first polarizing element 11 disposed to overlap with the first wavelength plate 12 and/or the second wavelength plate 17 is revolvable with respect to the second polarizing element 16 disposed to overlap with the second wavelength plate 17.

## Description

### TECHNICAL FIELD

The present invention relates to an optical unit which can obtain an image from which glare is removed when various objects are irradiated with light to obtain an image formed by reflection light from the objects.

### BACKGROUND ART

A flaw, stain, or the like (hereinafter collectively referred to as an "abnormal thing") may exist on a surface of various industrial products, foods, fresh produce, or the like (hereinafter collectively referred to as an "object"). It is necessary to find and eliminate such an abnormal thing in a manufacturing stage, a processing stage, an inspection stage, or the like. Usually, an abnormal thing is detected in such a manner that, to allow the abnormal thing to be easily visually recognized, an object is irradiated with light, and reflection light from the object is visually observed. In this case, in an inspection method where an object is irradiated with light and reflection light is detected, glare may be generated due to surface reflection of light, and this glare prevents abnormal things from being found. Accordingly, it is necessary to effectively remove glare.

An applicant of the present invention has proposed a head mounted lighting device which includes a light source unit and a transmission member disposed in front of the eyes (Patent Document 1). The head mounted lighting device is configured such that a first polarizing element, which converts emitted light to linearly polarized light, is provided to the light source unit and a second polarizing element, which has a polarization axis 90° orthogonal to the polarization axis of the first polarizing element, is provided to the transmission member to allow a wearer to visually recognize the surface of an object with no glare. Although Patent Document 1 proposes to use the head mounted lighting device mainly for medical purposes, Patent Document 1 also discloses that the head mounted lighting device can also be used as an industrial inspection device that finds an abnormal thing on the surface of an object.

Patent Document 1 also discloses that a wavelength plate is made to overlap with each of the first polarizing element and the second polarizing element, thus removing glare generated by a dynamic object, such as adhesion of water on an object.

### CITATION LIST

### PATENT DOCUMENT

Patent Document 1: Japanese Patent No. 6185686

### SUMMARY OF THE INVENTION

### PROBLEM TO BE SOLVED BY THE INVENTION

In detecting whether an abnormal thing exists or not on an object, as described above, the polarizing elements are respectively disposed on a light emission side and a light-receiving side such that polarization axes of the polarizing elements are directed to intersect at 90°, and the wavelength plate having the same configuration is disposed to overlap with each polarizing element and hence, glare can be effectively removed and existence of an abnormal thing can be easily viewed. That is, light which is linearly polarized light is caused to transmit through the wavelength plate to convert the light to circularly polarized light/elliptically polarized light in a clockwise direction/counterclockwise direction, and the object is irradiated with the circularly polarized light/elliptically polarized light. Further, reflection light from the object is also caused to transmit through a similar wavelength plate. With such a configuration, it is possible to obtain an image from which glare is removed effectively according to color or the condition of unevenness (the kind of abnormal thing), for example, of the object to be inspected.

When various objects are inspected by using one inspection device, an image from which glare is effectively removed can be obtained from one object. However, when a different object is inspected, there may be a case where glare is not sufficiently removed. The reason is considered to be as follows. The respective objects have different surface states and colors and hence, the respective objects differ in the manner of scattered reflection, color absorption, or reflection.

The present invention has been made to solve the above-mentioned problem, and it is an object of the present invention to provide an optical unit which can obtain an image from which glare is effectively removed regardless of the surface state of an object in detecting reflection light from the object by irradiating the object with light.

### MEANS FOR SOLVING PROBLEM

To achieve the above-mentioned object, the present invention is directed to an optical unit that irradiates an object with light and receives reflection light from the object to allow a surface state of the object to be visually recognized, the optical unit including: a light source configured to irradiate the object with light; a first polarizing element configured to convert emitted light emitted from the light source to linearly polarized light; a second polarizing element configured to cause the reflection light from the object to transmit through the second polarizing element, a polarization axis of the second polarizing element being 90° orthogonal to a polarization axis of the first polarizing element; a first wavelength plate disposed to overlap with the first polarizing element and configured to convert the emitted light from the light source to circularly polarized light or elliptically polarized light; and a second wavelength plate disposed to overlap with the second polarizing element, a phase difference of the second wavelength plate being equal to a phase difference of the first wavelength plate, wherein the first wavelength plate is revolvable with respect to the first polarizing element disposed to overlap with the first wavelength plate and/or the second wavelength plate is revolvable with respect to the second polarizing element disposed to overlap with the second wavelength plate.

In the above-mentioned optical unit, the first polarizing element and the second polarizing element are disposed such that the polarization axes of the first polarizing element and the second polarizing element are orthogonal to each other, the first wavelength plate is disposed to overlap with the first polarizing element, and the second wavelength plate having the phase difference equal to the phase difference of the first wavelength plate is disposed to overlap with the second polarizing element. With such a configuration, reflection light from the object is brought into a state where glare is removed and hence, the object can be easily visually recognized. In this case, when an object which is irradiated with light changes, the surface state (reflection state, light absorption rate or light reflectance, contrast) of the object changes. Accordingly, when different objects are visually recognized with one optical unit, light in some wavelength bands is reflected or absorbed on the surface of the object depending on the object. Therefore, color information of light is lost, and reflection light may be viewed in a hazy state (opaque state) (contrast varies). However, the first wavelength plate and/or the second wavelength plate are/is revolved to adjust intensity of light which passes through the second polarizing element to a minimum level, and a direction of the polarization and a direction of one axis of the wavelength plate are aligned (the wavelength plate is revolved to detect an angle having a large effect). With such operations, contrast is increased and hence, even various objects can be clearly visually recognized with one optical unit by removing glare.

For example, the above-mentioned optical unit can be used in an inspection device that inspects whether an abnormal thing exists on the surface of the object when objects are continuously conveyed, or can be used in an inspection device that inspects the surfaces of various objects in a state of being held by the hand or in a state of being mounted on the head. Further, the above-mentioned optical unit may be incorporated, as a constitutional element, in an observation device that observes a specific object, for example, an observation device (various medical devices) used for observing and diagnosing the pharynx in the oral cavity of the human body.

### EFFECT OF THE INVENTION

According to the present invention, it is possible to obtain an optical unit which can obtain an image from which glare is effectively removed regardless of the surface state of an object in irradiating the object with light to detect reflection light from the object.

### BRIEF DESCRIPTION OF DRAWINGS

Fig. 1 is a perspective view showing a first embodiment of an optical unit according to the present invention, and showing one example of an inspection device in which the optical unit is incorporated.
Fig. 2 shows the optical unit shown in Fig. 1, wherein Fig. 2(a) is a front view, Fig. 2(b) is a bottom view, and Fig. 2(c) is a side view.
Fig. 3 is an exploded perspective view showing constitutional elements of the optical unit shown in Fig. 2.
Fig. 4 is an exploded perspective view showing an incorporated state of optical element portions of the optical unit shown in Fig. 2.
Fig. 5 is an exploded perspective view of the optical element (polarizing elements, wavelength plates) portions of the optical unit shown in Fig. 4.
Fig. 6 is a block diagram showing a schematic configuration of the inspection device.
Fig. 7 is a photocopy of a photograph obtained in such a manner that a casing of a keyboard part of a notebook computer is irradiated with light by using the optical unit shown in Fig. 1, and reflection light from the casing is photographed with a camera (before glare is removed).
Fig. 8 is a photocopy of a photograph obtained in such a manner that a wavelength plate of a lighting-side unit is revolved by a predetermined amount from a state shown in Fig. 7, and reflection light from the casing is photographed with the camera (after glare is removed).
Fig. 9 is a photocopy of a photograph obtained in such a manner that a control board is irradiated with light by using the optical unit shown in Fig. 1, and reflection light from the control board is photographed with the camera (before glare is removed).
Fig. 10 is a photocopy of a photograph obtained in such a manner that the wavelength plate of the lighting-side unit is revolved by a predetermined amount from a state shown in Fig. 9, and reflection light from the control board is photographed with the camera (after glare is removed).
Fig. 11 is a photocopy of a photograph obtained in such a manner that a golf ball is irradiated with light by using the optical unit shown in Fig. 1, and reflection light from the golf ball is photographed with the camera (before glare is removed).
Fig. 12 is a photocopy of a photograph obtained in such a manner that the wavelength plate of the lighting-side unit is revolved by a predetermined amount from a state shown in Fig. 11, and reflection light from the golf ball is photographed with the camera (after glare is removed).
Fig. 13 is a photocopy of a photograph obtained in such a manner that an iron ball is irradiated with light by using the optical unit shown in Fig. 1, and reflection light from the iron ball is photographed with the camera (before glare is removed).
Fig. 14 is a photocopy of a photograph obtained in such a manner that the wavelength plate of the lighting-side unit is revolved by a predetermined amount from a state shown in Fig. 13, and reflection light from the iron ball is photographed with the camera (after glare is removed).
Fig. 15 is a cross-sectional view showing a second embodiment of the optical unit according to the present invention.
Fig. 16 is a view showing a third embodiment of the optical unit according to the present invention.
Fig. 17 is a view showing a fourth embodiment of the optical unit according to the present invention, and is also a perspective view showing configurations of a second wavelength plate and a second polarizing element held by a support member which is mounted on the head.
Fig. 18 is a partial cross-sectional view showing a configuration of a synchronization mechanism of the optical unit shown in Fig. 17.
Fig. 19 is a perspective view showing a fifth embodiment of the optical unit according to the present invention.
Fig. 20 is a view showing one example of a disposable cover which is detachably mounted on the optical unit shown in Fig. 19.

### MODE(S) FOR CARRYING OUT THE INVENTION

Hereinafter, embodiments of an optical unit according to the present invention will be described with reference to attached drawings.

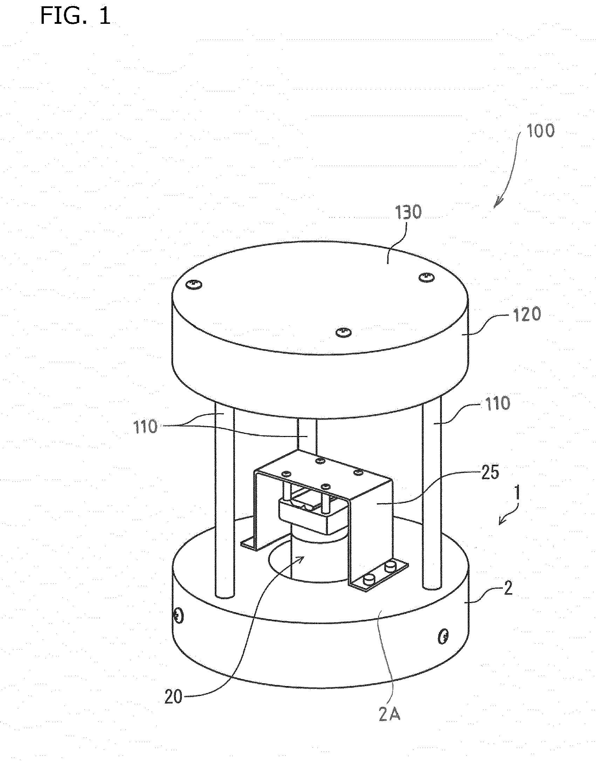

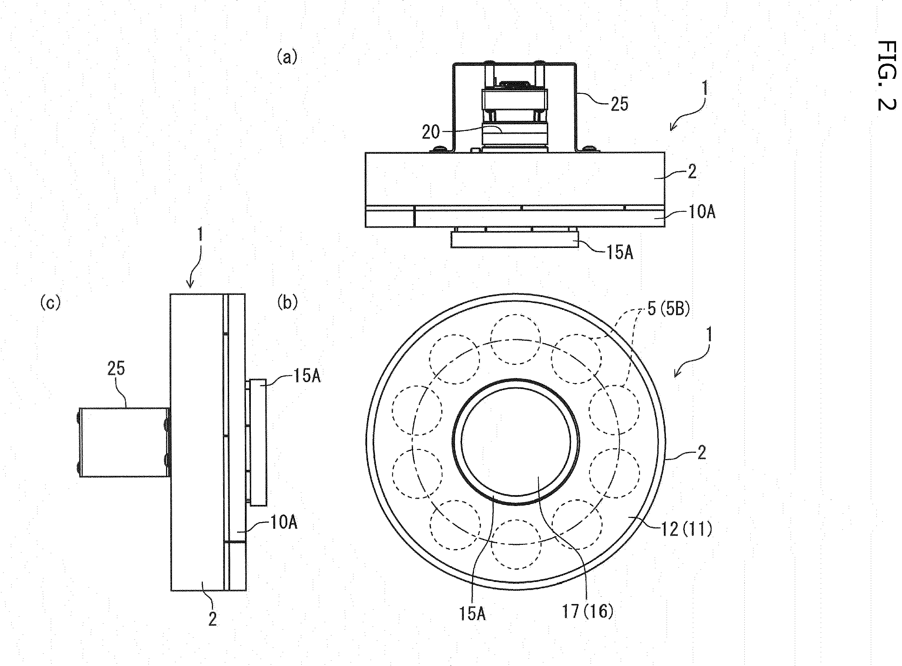

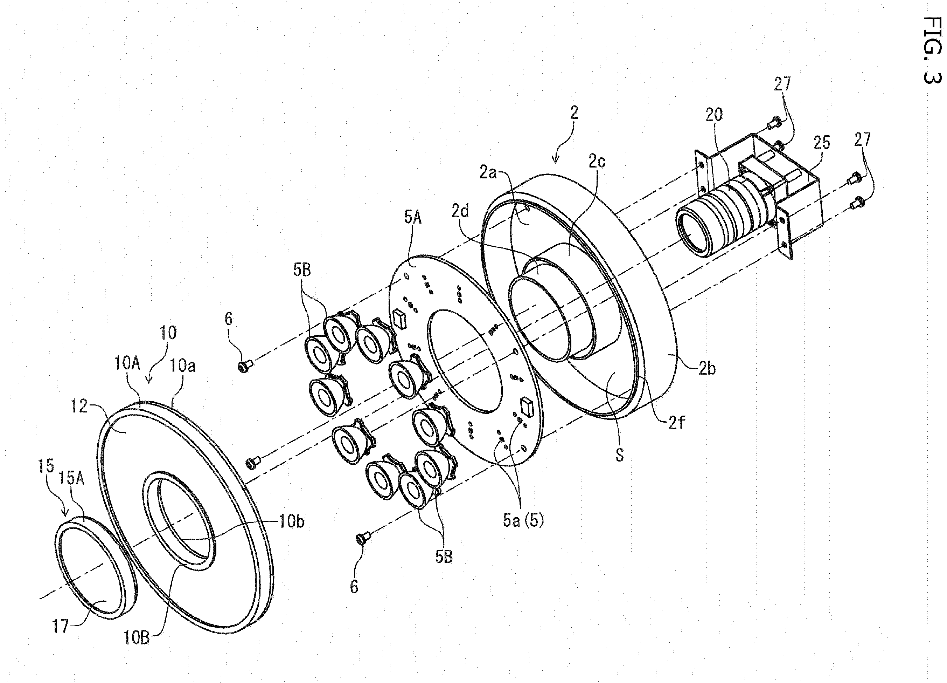

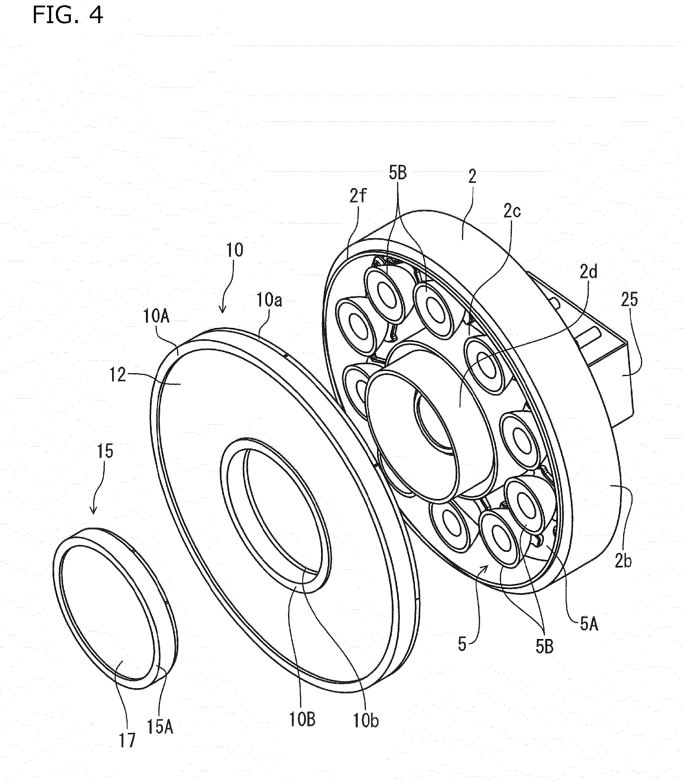

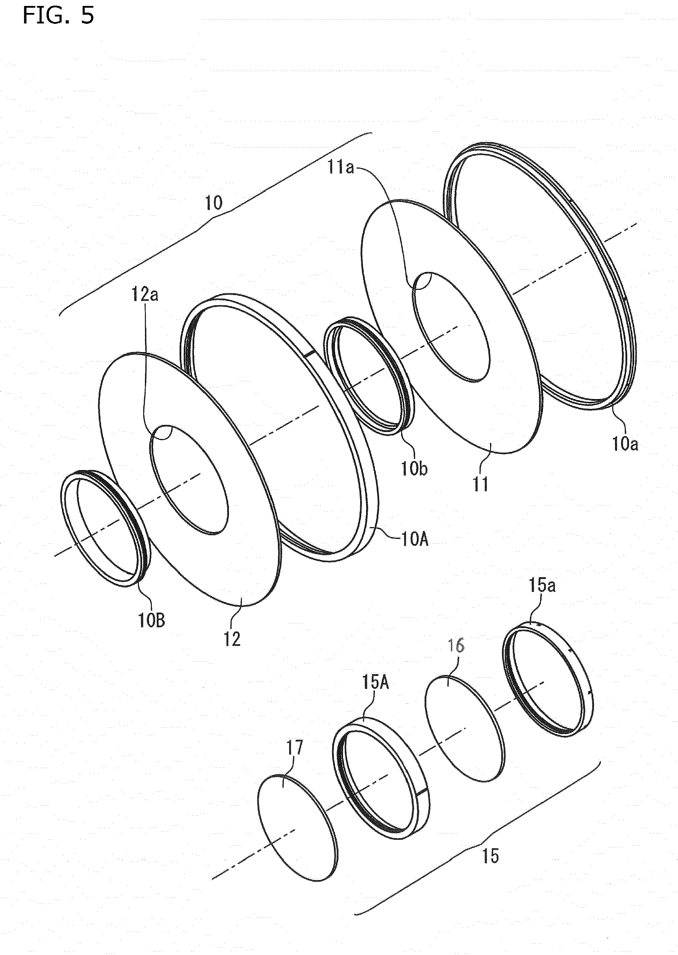

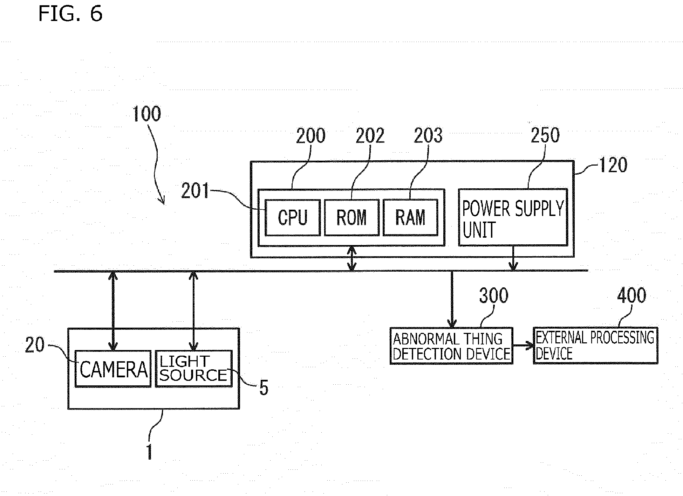

Fig. 1 to Fig. 6 are views showing a first embodiment. Fig. 1 is a perspective view showing one example of an inspection device in which an optical unit is incorporated. Fig. 2 shows the optical unit shown in Fig. 1, wherein Fig. 2(a) is a front view, Fig. 2(b) is a bottom view, and Fig. 2(c) is a side view. Fig. 3 is an exploded perspective view showing constitutional elements of the optical unit shown in Fig. 2. Fig. 4 is an exploded perspective view showing an incorporated state of optical element portions of the optical unit shown in Fig. 2. Fig. 5 is an exploded perspective view of the optical element portions of the optical unit shown in Fig. 4. Fig. 6 is a block diagram showing a schematic configuration of the inspection device.

An optical unit 1 of this embodiment can be incorporated in an inspection device 100 that is installed on and fixed to a conveyance path, through which objects, such as food or industrial products, are continuously conveyed, and that irradiates the object with light and receives reflection light from the object, thus detecting an abnormal thing existing on the object.

The optical unit 1 includes a casing having a cylindrical shape (in this embodiment, a casing formed into a cylindrical shape) 2. The casing 2 is provided with a light source and an image obtaining device (camera) 20, the light source irradiating, with light, objects that are continuously conveyed, the camera 20 receiving reflection light from the object, thus enabling detection of whether an abnormal thing exists or not on the object. The casing 2 is coupled with a cylindrical base 120 via a plurality of connecting rods 110, the base 120 having a shape substantially equal to the shape of the casing 2. The base 120 is fixed at a predetermined position of a facility. That is, the optical unit 1 of this embodiment is configured to be supported in a state of being suspended from the ceiling. One end of each connecting rod 110 is threadedly engaged with and fixed to the casing 2, and the other end of each connecting rod 110 is fixed to the base 120 formed into a cylindrical shape. The optical unit is not limited to the configuration that is supported in a state of being suspended from the ceiling, but may have a configuration that is supported by a stand.

The base 120 accommodates various functional components, such as a control unit (control board) 200 and a power supply unit 250. The control unit (control board) 200 includes a CPU 201, a ROM 202, and a RAM 203, the CPU 201 controlling actions of the functional components, such as a light source 5 and the camera 20 installed in the optical unit 1, the ROM 202 storing a control program for controlling actions of various drive elements, the RAM 203 temporarily storing various kinds of information according to the actions of the control program. The power supply unit 250 supplies power to the optical unit 1 and a control unit 200. A closing lid 130 is fitted on the base 120 to close the inside of the base 120.

The casing 2 is formed as a cylindrical case including a ring band-like bottom portion 2a and an annular wall 2b. An annular protrusion 2c is formed at the center portion of the bottom portion 2a. A portion surrounded by the bottom portion 2a, the annular wall 2b, and the annular protrusion 2c defines an accommodating portion S, and a circular ring case cover lid 2A is fixed to a rear side of the casing 2. A cylindrical annular protrusion 2d is formed at the center of the ring case cover lid 2A, and is brought into surface contact with the inner surface of the annular protrusion 2c, which is formed at the center portion of the bottom portion 2a of the casing 2. The ring case cover lid 2A and the annular protrusion 2d are members having a function of radiating heat. The annular protrusion 2d has a function of radiating heat of the annular protrusion 2c in a state where the ring case cover lid 2A is fixed to the casing 2.

A support portion (a support portion formed into a bridge shape) 25 in a state of holding the camera 20 is fixed to the center portion of the ring case cover lid 2A by set screws 27. The camera 20 attached to the support portion 25 is located in the annular protrusion 2d (2c), and is positioned such that the camera 20 receives reflection light from an object, the reflection light passing through a second polarizing element, which will be described later. That is, an image pickup element of the camera 20 receives reflection light from objects that are continuously conveyed, and an image signal obtained by the image pickup element is sent to an abnormal thing detection device 300 via the control unit. The abnormal thing detection device 300 can detect the presence or absence of an abnormal thing with respect to the received image signal with a predetermined detection program, and executes processing of removing the object on which an abnormal thing exists from a conveying line. For example, by sending an abnormal thing detection signal to an external processing device (object removal device) 400 installed on the downstream side of an object conveyance path, it is possible to execute processing, such as removing an object on which an abnormal thing exists from the conveying line.

The light source 5 is formed into a ring shape to surround the annular protrusion 2c, and is disposed on the bottom portion 2a. It is sufficient that the light source 5 can irradiate, with spot light or diffused light, objects that are continuously conveyed. In this embodiment, the light source 5 is formed of LED elements 5a disposed on a circumference at fixed intervals. These LED elements 5a are mounted on an LED board 5A formed into a ring shape. The LED board 5A is fixed to the bottom portion 2a by set screws 6.

A lens 5B is disposed to be positioned on each LED element 5a. Each lens 5B causes LED light emitted from each LED element 5a to be condensed with high illuminance at a predetermined position, specifically, over the width of the object conveying line within a range of a fixed light field diameter. The lens 5B in this embodiment has a function of condensing light emitted from the LED element and of irradiating an object with the light. Light may be condensed by one lens. Alternatively, as described above, by taking into account that light is condensed with high illuminance at a position with a fixed treatment distance and within a range of a fixed light field diameter, it is desirable to adopt a configuration that a lens which causes LED light to be condensed is disposed on each of a plurality of LED elements, and light from the respective lens is condensed.

Alternatively, provided that fixed illuminance is ensured for an object, light from the light source 5 may be diffused light (including parallel light), and the lens may adopt any of various configurations. That is, provided that a lens system is configured to irradiate the object with spot light, parallel light, or diffused light which is emitted from the light source, a configuration of the lens system is not particularly limited. The light source 5 may be an organic EL light source or a surface emitting laser, for example, so as to uniformly irradiate a large area, or may be an electrodeless lamp or the like.

A lighting-side unit (lighting-use phase difference ring) 10 is disposed on the opening edge of the casing 2 so as to be positioned in front of the lenses 5B, and a camera-side unit (camera-use phase difference ring) 15 is disposed in front of the annular protrusion 2d. The lighting-side unit 10 converts emitted light emitted from the ring-shaped light source 5 to a polarization state (circularly polarized light or elliptically polarized light) and thus irradiating the object with the light in the polarization state. The camera-side unit 15 converts reflection light (circularly polarized light or elliptically polarized light) from an object to a linearly polarized light, and causes the camera 20 to receive the linearly polarized light. That is, the lighting-side unit 10 is formed into a ring shape, and the camera-side unit 15 is formed into a disk shape so as to be disposed inward of the lighting-side unit 10 in the radial direction and hence, a layout is allowed where the lighting-side unit and the camera-side unit can be disposed in a compact manner.

Hereinafter, configurations of the lighting-side unit 10 and the camera-side unit 15 will be described.

The lighting-side unit 10 includes an outer peripheral side fixing ring 10a, an inner peripheral side fixing ring 10b, and a first polarizing element 11. The outer peripheral side fixing ring 10a is fixed to an opening edge 2f of the casing 2. The inner peripheral side fixing ring 10b is fixed to the outer peripheral surface of the annular protrusion 2d. The first polarizing element 11 is held and fixed between the respective rings. The first polarizing element 11 is formed into a circular shape having an opening 11a at the center so as to be fixed to the inner peripheral edge of the outer peripheral side fixing ring 10a and so as to be fixed to the outer peripheral edge of the inner peripheral side fixing ring 10b. The first polarizing element 11 has a function of converting emitted light emitted from the ring-shaped light source 5 to linearly polarized light.

The lighting-side unit 10 also includes an outer peripheral side movable ring 10A, an inner peripheral side movable ring 10B, and a first wavelength plate 12. The outer peripheral side movable ring 10A is disposed in a juxtaposed manner with the outer peripheral side fixing ring 10a, and is held in a revolvable manner in the circumferential direction. The inner peripheral side movable ring 10B is disposed in a juxtaposed manner with the inner peripheral side fixing ring 10b, and is held in a revolvable manner in the circumferential direction. The first wavelength plate 12 is held and fixed between the respective rings. The first wavelength plate 12 is formed into a circular shape having an opening 12a at the center so as to be fixed to the inner peripheral edge of the outer peripheral side movable ring 10A and so as to be fixed to the outer peripheral edge of the inner peripheral side movable ring 10B. The first wavelength plate 12 has a function of converting linearly polarized light which transmits through the first polarizing element 11 to circularly polarized light or elliptically polarized light in a clockwise direction/counterclockwise direction. By gripping the outer peripheral side movable ring 10A and performing revolving operation, the first wavelength plate 12 can be revolved through 360°.

The camera-side unit 15 includes a fixing ring 15a and a movable ring 15A. The fixing ring 15a is fixed to the front end side of the annular protrusion 2d. The movable ring 15A is disposed in a juxtaposed manner with the fixing ring 15a and is held by the fixing ring in a revolvable manner in the circumferential direction. The camera-side unit 15 is disposed inward of the ring-shaped light source in the radial direction. The camera-side unit 15 also includes a second polarizing element 16 and a second wavelength plate 17. The second polarizing element 16 is held by and fixed to the fixing ring 15a. The second wavelength plate 17 is held by and fixed to the movable ring 15A. In this case, the camera-side unit 15 is disposed in a state of protruding from the lighting-side unit 10 in the axial direction, thus allowing the movable ring 15A to be gripped for performing revolving operation of the movable ring 15A. That is, by gripping the movable ring 15A and performing revolving operation, the second wavelength plate 17 can be revolved through 360°.

The second polarizing element 16 is held by and fixed to the fixing ring 15a such that the second polarizing element 16 has a polarization axis which is 90° orthogonal to (including an angle substantially orthogonal to) the polarization axis of the first polarizing element 11. A wavelength plate having the same phase difference as the first wavelength plate 12 is used for the second wavelength plate 17. The first wavelength plate 12 is disposed to overlap with the first polarizing element 11. The second wavelength plate 17 is disposed to overlap with the second polarizing element 16.

In the optical unit 1 having the above-mentioned configuration, light emitted from the ring-shaped light source 5 is changed to linearly polarized light by the first polarizing element 11. That is, the first polarizing element 11 converts light (light in a non-polarization state) emitted from the light source 5 to linearly polarized light, which has a fixed oscillation direction of an electric field (and a magnetic field), and objects that are continuously conveyed are irradiated with this linearly polarized light. In this case, if the surface of the object is in a mirror surface state, reflection light from the object forms linearly polarized light without any conversion. However, an abnormal thing, such as unevenness, is present on an actual object and hence, reflection light forms diffused light (in a non-polarization state) containing components of linearly polarized light. As described above, reflection light from the object is diffused and reflected and hence, even if incident light is linearly polarized light, reflection light from the object is brought into a non-polarization state. This light in the non-polarization state contains components of a specular reflection light (an incident linearly polarized light forms linearly polarized light without any conversion, and is reflected). The components of the specular reflection light form glare of the light source, thus preventing determination of the presence of an abnormal thing.

That is, an abnormal thing is detected mainly based on variation in contrast and hence, if glare is generated, it becomes difficult to find the abnormal thing. However, the camera 20 supported by the casing 2 detects reflection light which transmits through the second polarizing element 16 having the polarization axis 90° orthogonal to the polarization axis of the first polarizing element 11. Accordingly, the camera 20 can obtain an image of diffused and reflection light from which reflection components which become a cause of glossiness are removed and hence, an object image signal sent from the camera 20 is a signal for a surface state with no glare whereby it is possible to easily determine the presence of an abnormal thing.

Each of the above-mentioned first polarizing element 11 and the second polarizing element 16 may be formed of a linearly polarizing filter, a film polarizer, a nano wire grid polarizing plate, an inorganic polarizing plate (a plate obtained in such a manner that a thin aluminum film is formed on a glass wafer, and minute slits are formed on the thin aluminum film) or the like, for example. Alternatively, each of the above-mentioned first polarizing element 11 and the second polarizing element 16 may be configured such that a thin film having such a function is formed on the element.

Further, in the above-mentioned configuration, emitted light from the light source 5 may be electrically subjected to frequency modulation (pulse modulation) by an external signal to vary phase, amplitude, and a polarization plane, thus accurately aligning an oscillation direction of the electric field of light (an oscillation direction of the electric field of light is brought into an aligned state). Then, the object may be irradiated with this light through a wire grid polarizing plate forming the first polarizing element having high transmittance. Also with such a configuration, glare can be removed effectively.

In the optical unit 1 having the above-mentioned configuration, it is preferable that the first wavelength plate (a 1/2 wavelength plate, a 1/4 wavelength plate, a 1/8 wavelength plate, or the like) 12 be disposed to overlap with the first polarizing element 11. It is preferable that the second wavelength plate 17 having a phase difference equal to a phase difference of the first wavelength plate 12 be disposed to overlap with the second polarizing element 16.

In general, in obtaining reflection light by irradiating an object with light, when a polarized light where the oscillation direction of the electric field of the light is accurately aligned is used, a polarized light where the oscillation direction of the electric field of the light is accurately aligned is reflected from the object. In this case, when the object is irradiated with circularly polarized light or elliptically polarized light and time required for transmission of the light and variation in intensity of the light are observed, it is possible to obtain information of chirality, which is a difference in a three-dimensional arrangement of molecules. Usually, the surface of the object has various states and hence, the surface cannot specularly reflect light, thus causing scattered reflection. Further, emitted light in some wavelength bands is reflected or absorbed depending on surface color and hence, color information of light is lost (contrast varies). Due to such factors, reflection light is viewed in a hazy state (opaque state).

As described above, due to factors, such as color of an object, the condition of unevenness on a surface, and adhesion of water, scattered reflection on the surface varies depending on the object and color information of light also varies widely. Accordingly, light which is linearly polarized light is caused to transmit through the above-mentioned wavelength plate 12 to convert the light to circularly polarized light/elliptically polarized light (in an appropriate polarization state) in the clockwise direction/counterclockwise direction, and the object is irradiated with the circularly polarized light/elliptically polarized light. Reflection light from the object is also caused to transmit through the wavelength plate 17 having the same phase difference as the wavelength plate 12. With such a configuration, glare can be removed more effectively. That is, the manner of viewing object varies, and the reflection angle of light also varies depending on the state of a flaw or dust, background color, and the like. Accordingly, by revolving the wavelength plate to change a phase angle, it is possible to obtain a state where the light can be easily viewed.

The reason will be specifically described. For example, light has characteristics that when the light is reflected off a water surface or the like, the light is changed to light having large lateral oscillation. Accordingly, when reflection light is circularly polarized, longitudinal oscillation is extremely reduced, and the light is changed to light having oscillation almost only in the lateral direction. Therefore, when reflection light is converted to linearly polarized light after being converted to circularly polarized light, glare can be effectively reduced even if water adheres to the surface of the object. That is, according to color or the condition of unevenness of the object to be inspected (the kind of abnormal thing), for example, the first wavelength plate 12 and the second wavelength plate 17 which are made to overlap with the above-mentioned polarizing element 11 and polarizing element 16 are brought into an appropriate polarization state according to the object. Accordingly, it is possible to obtain an image from which glare is removed more effectively.

In this embodiment, as described above, the first wavelength plate 12, which is made to overlap with the first polarizing element 11, is disposed such that the first wavelength plate 12 can be revolved with respect to the first polarizing element 11. The second wavelength plate 17, which is made to overlap with the second polarizing element 16, is disposed such that the second wavelength plate 17 can be revolved with respect to the second polarizing element 16. The reason is that the following fact is taken into consideration. The angle of reflection, absorption of light and the like vary depending on components of an abnormal thing (a flaw, dust, or the like), so that the manner of viewing reflection light changes. Revolving operation of the first wavelength plate 12 and/or the second wavelength plate 17 is performed to adjust intensity of light which passes through the second polarizing element 16 to a minimum level, and a direction of the polarization and a direction of one axis of the wavelength plate are aligned (the wavelength plate is revolved to detect an angle having a large effect). With such operations, contrast is increased and hence, an abnormal thing existing on the object can be made clearer without generating glare. In this case, either one of the first wavelength plate 12 or the second wavelength plate 17 may be revolved. However, it is preferable that the first wavelength plate 12, which is disposed on a light emission side, be brought into a fixed state, and the second wavelength plate 17, which is disposed on a light-receiving side, be revolved to detect reflection light. With such a configuration, when the light source on the light emission side is fixed in a state which does not generate specular reflection and the wavelength plate on the observation side is revolved, a position at which glare is effectively removed can be easily detected.

In layout of this embodiment, the wavelength plates 12, 17 are installed corresponding to the first polarizing element 11 and the second polarizing element 16, respectively. Therefore, for example, compared with a layout where a half mirror, a beam splitter and the like are disposed on an optical path and glare is removed by one wavelength plate, the layout of this embodiment has no variation in light quantity and a polarization state and hence, there is no possibility that the object cannot be easily viewed.

The position of each of the wavelength plates 12, 17 at which glare can be removed varies depending on a surface shape, color, contrast, and the like of the object and hence, a relationship between the object and the positions of the wavelength plates cannot be clearly specified. However, the description will be made with respect to images obtained in such a manner that various objects are actually irradiated with light by using the optical unit 1 having the above-mentioned configuration, and images of reflection light from the objects are obtained with the camera 20. In this embodiment, a plurality of objects which differ in surface state, such as brightness of reflection light from the surface of the object, contrast, and the state of unevenness, are prepared, and the respective objects are examined. A 1/4 wavelength plate is used for each of the first wavelength plate 12 and the second wavelength plate 17.

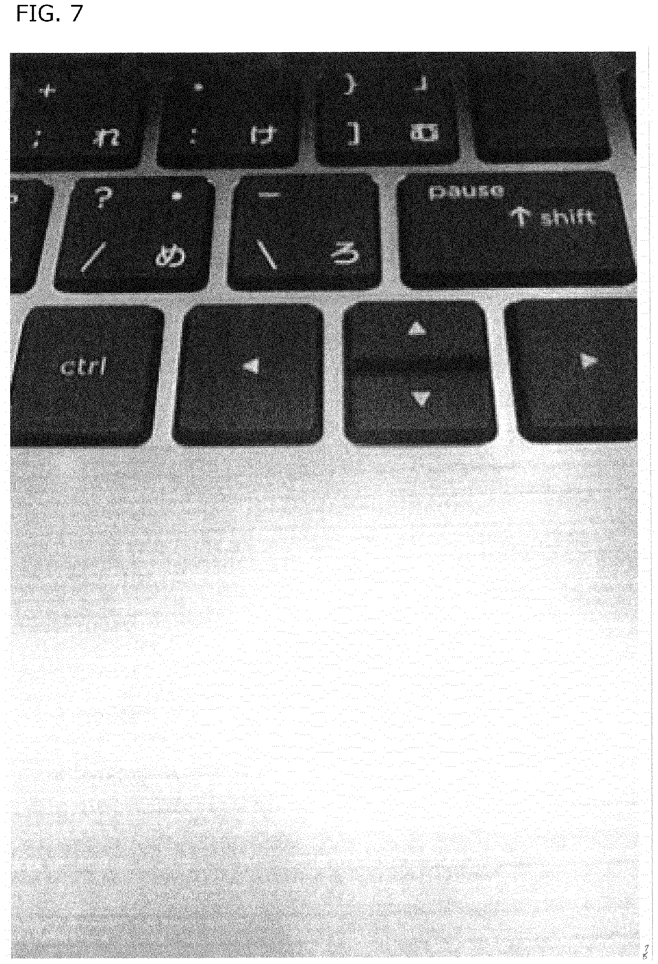

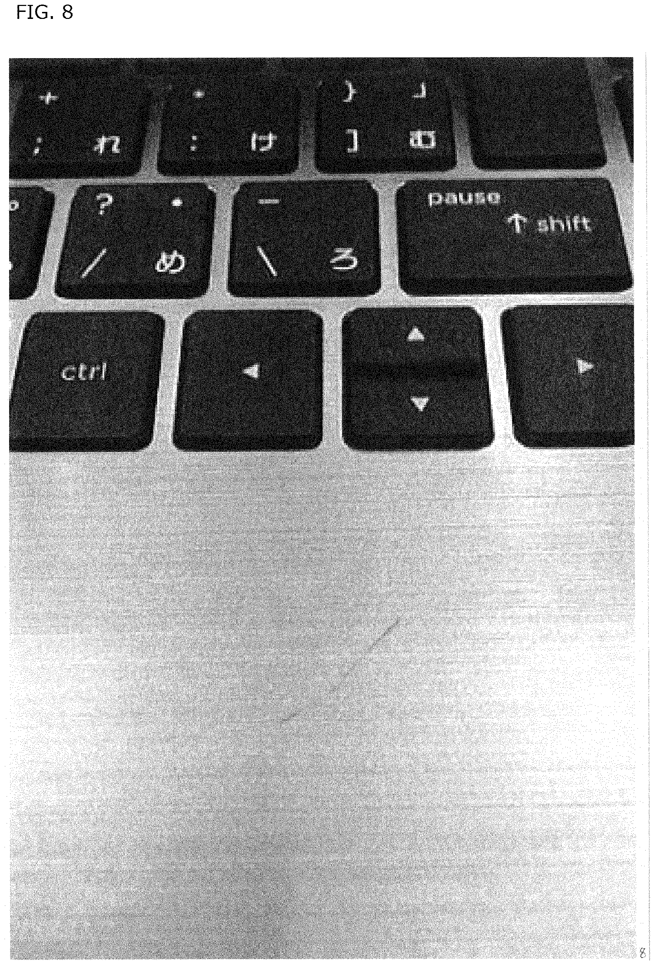

Fig. 7 is an image obtained in such a manner that a casing (brilliant silver color) of a keyboard part of a notebook computer is irradiated with light emitted from the light source, and reflection light from the casing is photographed with the camera 20. Fig. 7 shows a state where the light emitted from the light source is captured in the reflection light from the casing part, thus generating glare. When the outer peripheral side movable ring 10A of the lighting-side unit 10 is revolved approximately 1/8 revolution from such a state, the image shown in Fig. 8 can be obtained. In Fig. 8, glare at the casing part which is shown in Fig. 7 is removed and hence, a flaw existing on the surface of the casing part can be clearly visually recognized.

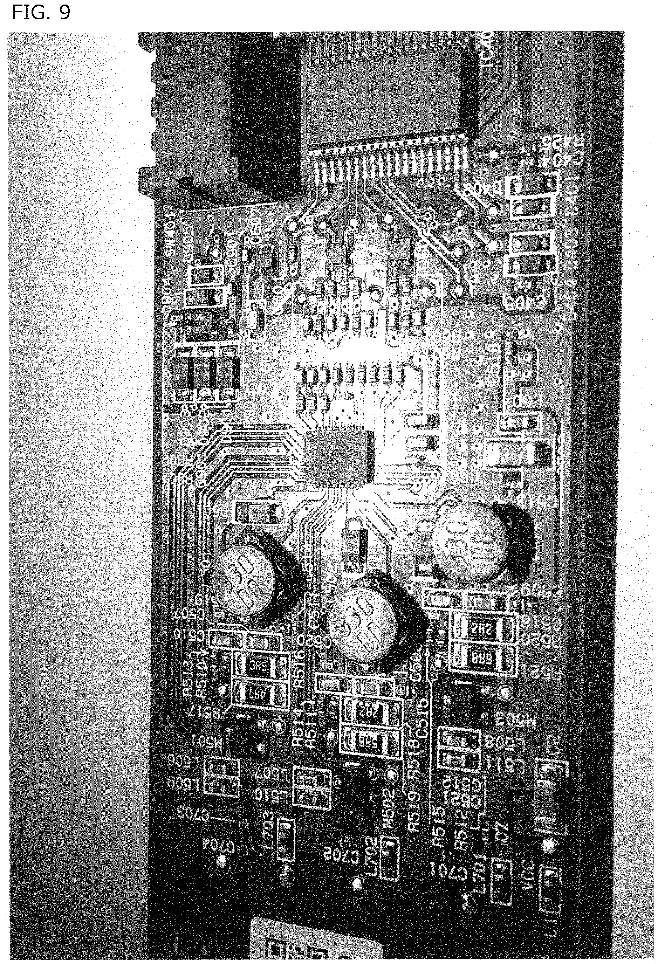

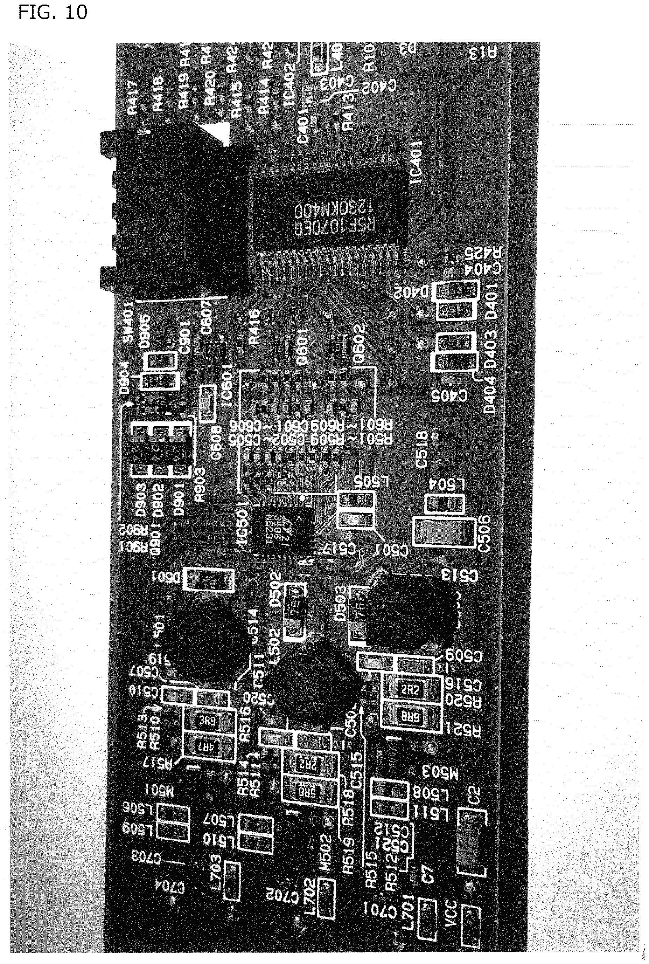

Fig. 9 is an image obtained in such a manner that a green control board on which various elements are mounted is irradiated with light emitted from the light source, and reflection light from the control board is photographed with the camera 20. Fig. 9 shows a state where glare is generated in the reflection light from a black CPU part at a center portion and a region of brilliant lead frames extending from a periphery of the CPU part. However, when the outer peripheral side movable ring 10A of the lighting-side unit 10 is revolved approximately 1/12 revolution from such a state, the image shown in Fig. 10 can be obtained. In Fig. 10, glare is removed from the entire control board and hence, mounting states of the respective elements can be clearly visually recognized.

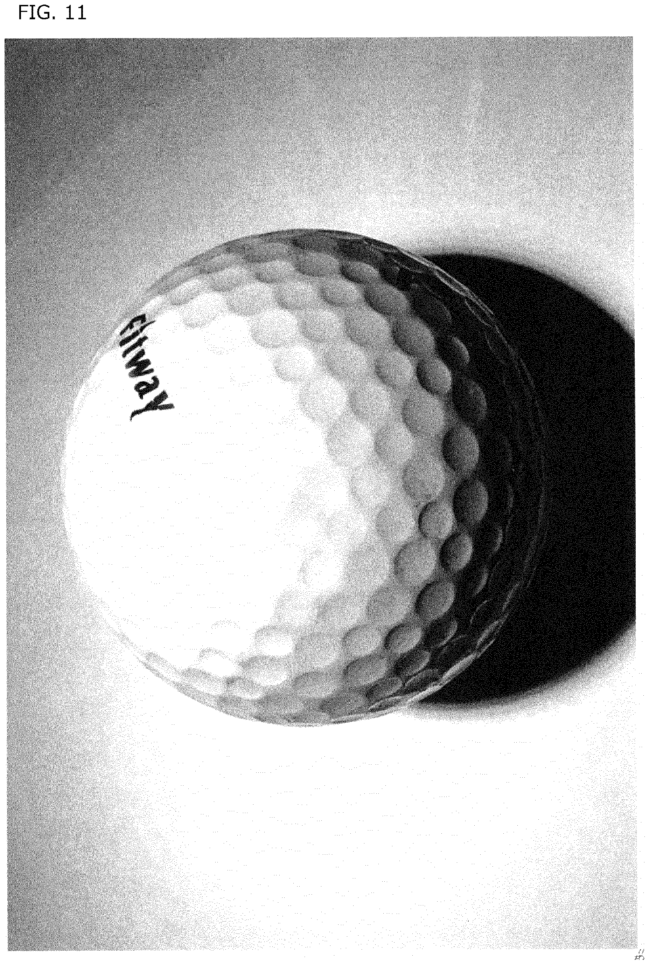

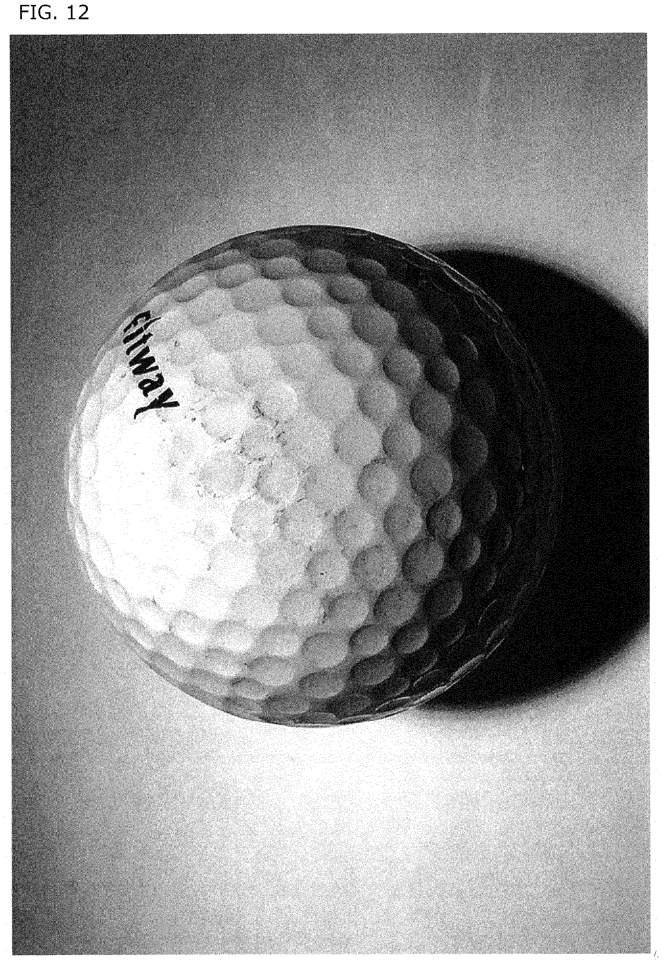

Fig. 11 is an image obtained in such a manner that a white golf ball is irradiated with light emitted from the light source, and reflection light from the golf ball is photographed with the camera 20. Fig. 11 shows a state where glare is generated in the reflection light from a top region of a spherical shape. However, when the outer peripheral side movable ring 10A of the lighting-side unit 10 is revolved approximately 1/10 revolution from such a state, the image shown in Fig. 12 can be obtained. In Fig. 12, glare in the top region is removed and hence, existence of stain on the surface of the golf ball can be clearly visually recognized.

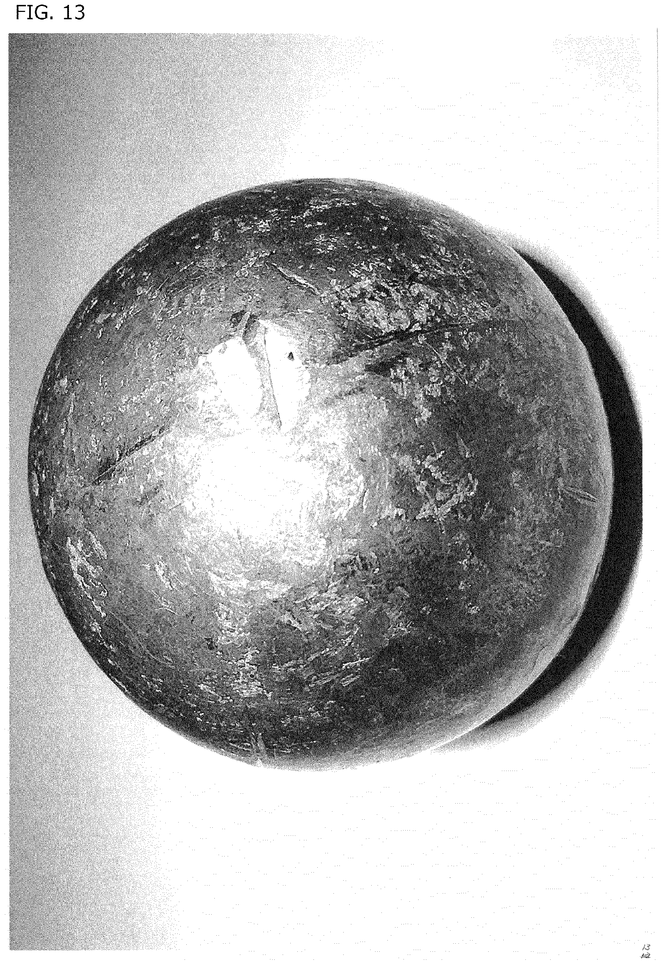

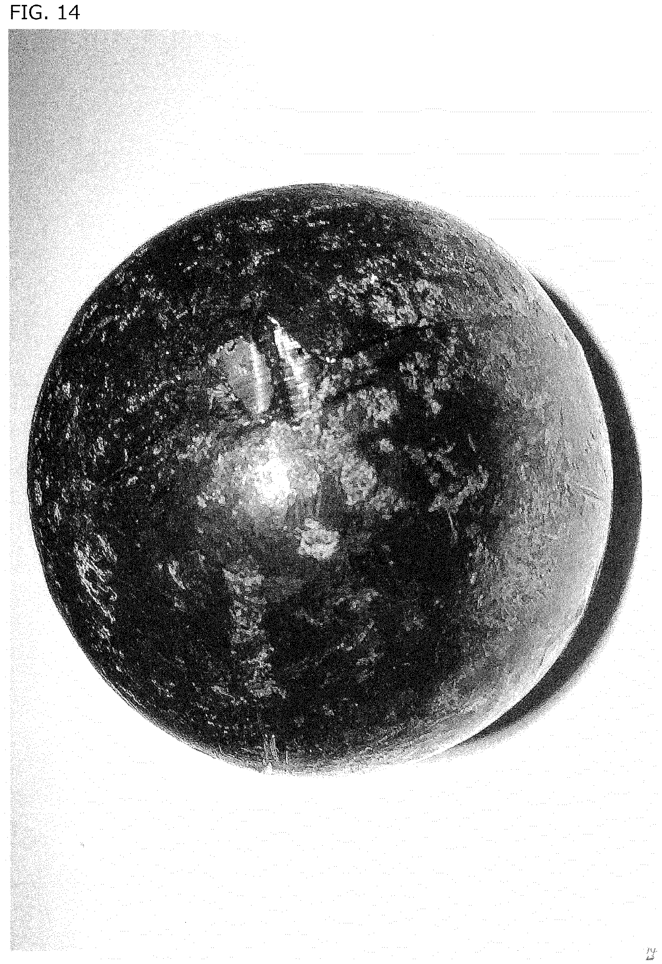

Fig. 13 is an image obtained in such a manner that an iron ball where a surface is in a rough state is irradiated with light emitted from the light source, and reflection light from the iron ball is photographed with the camera 20. Fig. 13 shows a state where glare is generated in the reflection light from the top region of the iron ball. However, when the outer peripheral side movable ring 10A of the lighting-side unit 10 is revolved approximately 1/12 revolution from such a state, the image shown in Fig. 14 can be obtained. In Fig. 14, glare in the top region is removed and hence, rust formed on the surface of the iron ball can be clearly visually recognized.

In addition to the above-mentioned objects, various other articles are also inspected. Even using any type of wavelength plate, when the wavelength plate is revolved at least 1/16 revolution or more, the image from which glare is removed (an image in which an abnormal thing can be clearly visually recognized) can be obtained. Therefore, it is preferable that the outer peripheral side movable ring 10A be mounted on the casing 2 such that the wavelength plate has a click at each 1/16 revolution. When the wavelength plate is stopped at a revolution position having a click, an image from which glare is removed can be obtained. The respective wavelength plates may be disposed such that only the wavelength plate 12 on the lighting side can be revolved, only the wavelength plate 17 on the camera side can be revolved, or both the wavelength plate 12 and the wavelength plate 17 can be revolved. It is sufficient to select an appropriate wavelength plate according to the kind of an object, the state of an abnormal thing and the like, and to revolve either one of the wavelength plate 12 or the wavelength plate 17. Needless to say, a configuration may be adopted where the wavelength plate has no clicks, thus revolving to continuously adjust (linearly adjust) the position of the wavelength plate. In addition to the configuration where revolving operation of the movable ring is performed, for example, a configuration may be adopted where a rack and pinion or the like is provided, and slide operation is performed to revolve the wavelength plate.

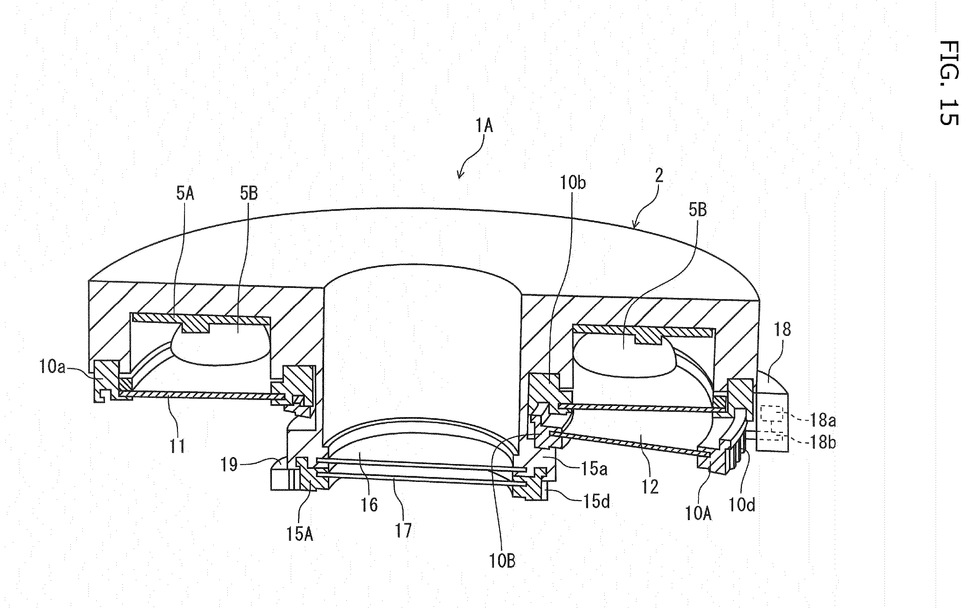

Fig. 15 is a cross-sectional view showing a second embodiment of the optical unit of the present invention.

The above-mentioned embodiment adopts the configuration where revolving operation of the wavelength plate 12 is performed by gripping the outer peripheral side movable ring 10A by the hand, and revolving operation of the wavelength plate 17 is performed by gripping the movable ring 15A by the hand. However, a configuration may be adopted where, as shown in Fig. 15, each of the wavelength plate 12 and the wavelength plate 17 is revolved and driven by drive means, such as a drive motor.

For example, an outer gear 10d is formed on the outer peripheral surface of the outer peripheral side movable ring 10A, and a gear box 18 is attached to the casing 2, the gear box 18 accommodating a drive motor 18a and a drive gear (may also be a gear train) 18b which is meshed with the outer gear 10d. With such a configuration, it is possible to revolve and drive the outer peripheral side movable ring 10A (wavelength plate 12). In this case, a control unit, which is installed in the above-mentioned base 120 (see Fig. 1), controls the driving of the drive motor 18a based on contrast information for the image obtained by the camera 20 such that the drive motor 18a is stopped when the image is in an optimum state. With such control, it is possible to always optimize the position of the wavelength plate 12. Accordingly, it is possible to obtain an image with no glare without being affected by the kind or state of an object, for example.

In the same manner, an outer gear 15d is formed on the outer peripheral surface of the movable ring 15A for the wavelength plate 17 disposed inward in the radial direction, and a gear box 19 is attached to the casing 2 (the fixing ring 15a), the gear box 19 accommodating a drive motor and a drive gear (not shown in the drawing) which is meshed with the outer gear. With such a configuration, it is also possible to revolve and drive the wavelength plate 17.

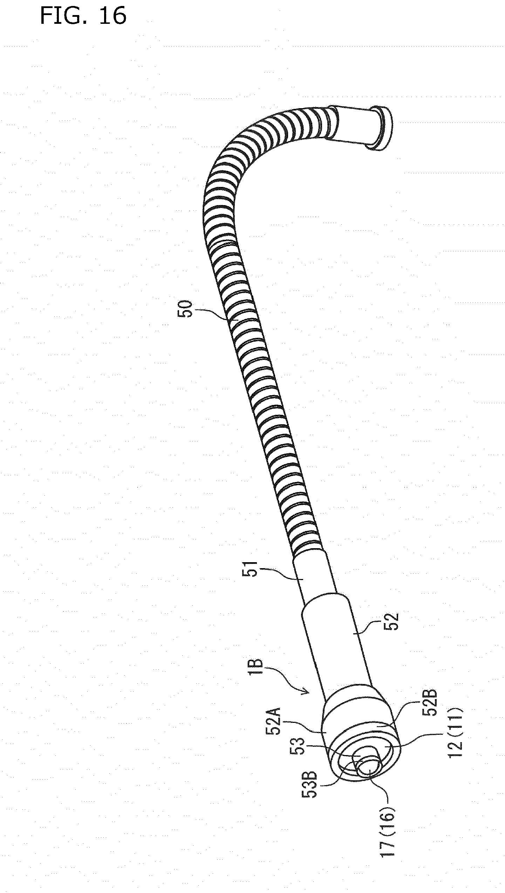

Fig. 16 is a view showing a third embodiment of the optical unit according to the present invention.

An optical unit 1B according to this embodiment is electrically and mechanically attached to a connection portion 51 which is provided to the end portion of a tubular body 50 formed to be flexible. A transmission cable, a power cable, and the like are disposed in the tubular body 50. Image signals from the camera (not shown in the drawing), which forms an image obtaining device disposed in the optical unit, are sent to an external device through the transmission cable. Power for activating the optical unit is supplied through the power cable.

Accordingly, with the optical unit 1B in this embodiment, it is possible to display an image in real time on a monitor installed outside the optical unit 1B. Therefore, by observing the monitor, a worker can perform inspection work by visually observing whether an abnormal thing exists on an object.

A casing 52 of the optical unit 1B is formed into a cylindrical shape (formed into a circular cylindrical shape in this embodiment) with a size that allows hand-held operation. An expanded diameter portion 52A is formed on the distal end side of the casing 52 to ensure a space for disposing a ring-shaped light source. A lens barrel 53 accommodating a camera is fixed inside the casing 52 along the direction of the center axis of the casing 52.

In the same manner as the above-mentioned embodiment,
an LED board on which LED elements are mounted is fixed to the expanded diameter portion 52A of the casing (a ring-shaped light source is formed) at a position outward of the lens barrel 53 in the radial direction. The LED elements are disposed on a circumference at fixed intervals. A ring-shaped first polarizing element 11 is fixed to the distal end side of the expanded diameter portion 52A, and a first wavelength plate 12 is disposed to overlap with the first polarizing element 11. In this case, when revolving operation of the movable ring 52B, which is revolvably supported by the expanded diameter portion 52A, is performed by gripping the movable ring 52B, the first wavelength plate 12 can be revolved integrally with the movable ring 52B.

A movable ring 53B is disposed on the distal end side of the lens barrel 53 at a position inward of the light source in the radial direction, the movable ring 53B holding the disk-shaped second wavelength plate 17. The second polarizing element 16 is fixed at a position inward of the movable ring 53B in the axial direction of the lens barrel 53.

In the same manner as the above-mentioned embodiment, the first polarizing element 11 has a function of converting emitted light emitted from the ring-shaped light source, which is disposed in the expanded diameter portion 52A, to linearly polarized light. The second polarizing element 16 is disposed such that the second polarizing element 16 causes reflection light from the object to pass therethrough and has the polarization axis which is 90° orthogonal to the polarization axis of the first polarizing element 11. Further, the first wavelength plate 12 is revolvably supported by the expanded diameter portion 52A such that the first wavelength plate 12 can be revolved with respect to the polarizing element 11. The second wavelength plate 17 is revolvably supported by the lens barrel 53 such that the second wavelength plate 17 can be revolved with respect to the polarizing element 16.

With such an optical unit 1B, the polarizing elements 11, 16 and the wavelength plates 12, 17 exhibit functions substantially equal to the functions in the first embodiment, and an image of reflection light obtained from the object can be displayed on an external monitor or the like with no glare. Accordingly, when a worker irradiates a predetermined portion of an inspection object with light from the light source while gripping the casing 52 and observes reflection light from the object, the worker can inspect an abnormal thing.

A device that obtains an image is not limited to the configuration where the camera is installed in the lens barrel 53, and may have a configuration where a fiberscope is used as the flexible tubular body 50 and an eyepiece or the like is attached to the end portion of the tubular body 50 to allow an image from an object to be directly and visually recognized. Alternatively, a configuration may be adopted where a magnifying glass is disposed in the lens barrel to allow an object to be observed in a magnified manner.

The optical unit in this embodiment can be operated by gripping the casing 52 by the hand. Accordingly, the optical unit can be used as an inspection device that inspects whether an abnormal thing exists on the rear side or at a narrow portion of any of various devices. Further, the optical unit can also be used as a medical device that examines the inside of the oral cavity. In the case where the optical unit is used as the medical device that examines the inside of the oral cavity, as will be described later in a fifth embodiment, it is preferable that a disposable cover (not shown in the drawing) is attached to a portion which is to be inserted into the oral cavity, the disposable cover being mounted on and removed from the portion for each patient.

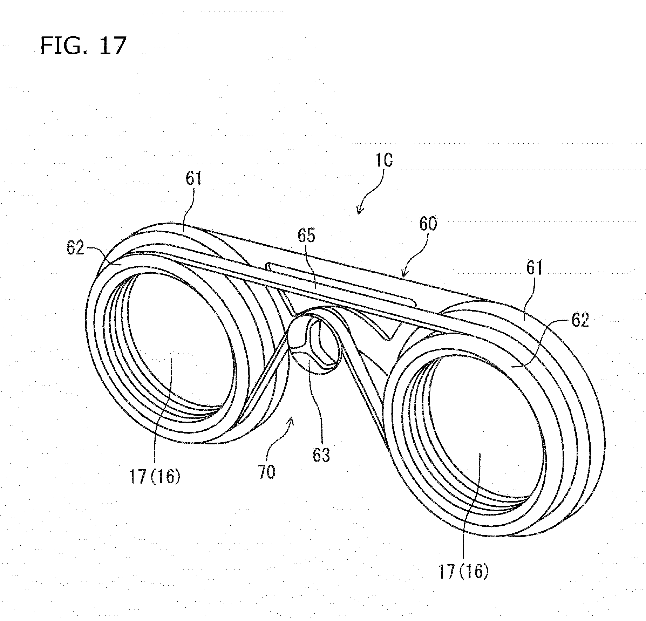

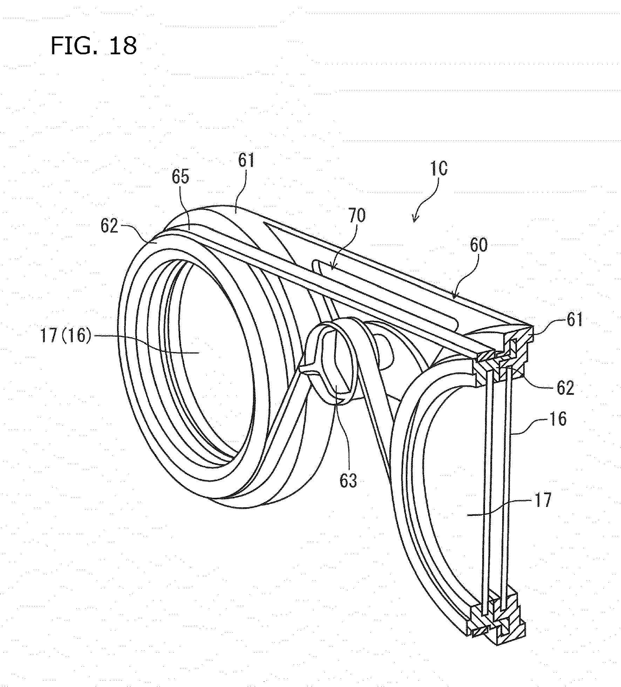

Fig. 17 and Fig. 18 are views showing a fourth embodiment of the optical unit according to the present invention. Fig. 17 is a perspective view showing a configuration of second wavelength plates and second polarizing elements held by a support member mounted on the head. Fig. 18 is a partial cross-sectional view showing a configuration of a synchronization mechanism.

The optical unit according to the present invention is not limited to a type which is installed in a facility or a hand-held type as described in the above-mentioned embodiments, and may be a head-mount-type optical unit 1C.

In this case, although not shown in the drawing in detail, a light source, first polarizing elements, and first wavelength plates which form the optical unit may be fixed to a mounting member (cap, glasses, belt, or the like) which is mounted on the head. For reflection light reflected from an object, it is necessary to dispose second polarizing elements and second wavelength plates in front of the left and right eyes. If the second wavelength plates, which are respectively disposed in front of the left and right eyes, are caused to be revolved, it is necessary to provide a synchronization mechanism that causes the second wavelength plates to revolve simultaneously.

A synchronization mechanism 70 in this embodiment is configured such that fixing rings 61, which are located in front of the left and right eyes, are provided to a support member 60, which is fixed at a position in front of the eyes, to fix the second polarizing elements 16, and a pair of movable rings 62 are revolvably supported on the fixing rings at positions in front of the fixing rings 61, each movable ring 62 holding the second wavelength plate 17. A coupling belt 65 is wound around both movable rings 62, and the coupling belt 65 is maintained in a tensioned state by a tension pulley 63, which is revolvably supported at a position between both movable rings 62.

In such a configuration, when revolving operation of either of movable rings 62 is performed by gripping one movable ring 62, the other movable ring is synchronously driven and hence, the second wavelength plates 17 can be integrally revolved and driven at positions in front of the left and right eyes.

The support member 60 including the above-mentioned synchronization mechanism 70 can be integrally attached to the mounting member (a cap, glasses, a belt, or the like which holds the light source, the first polarizing elements, and the first wavelength plates) which is mounted on the head. When a wearer revolves the second wavelength plate 17, the wearer can visually recognize reflection light from an object with no glare.

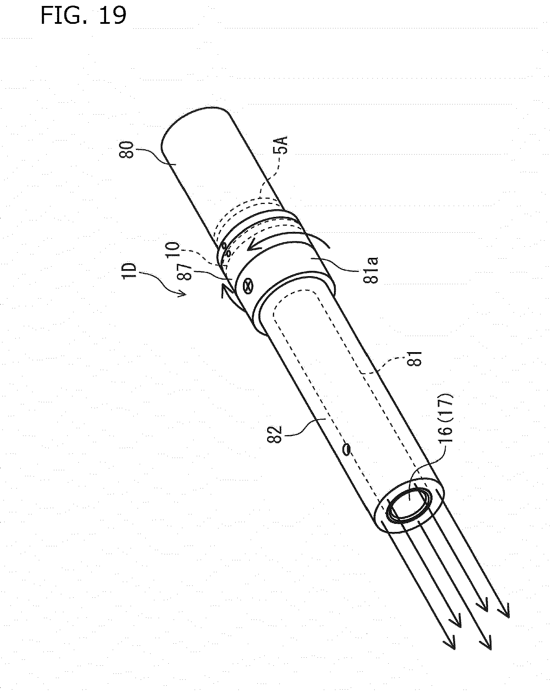

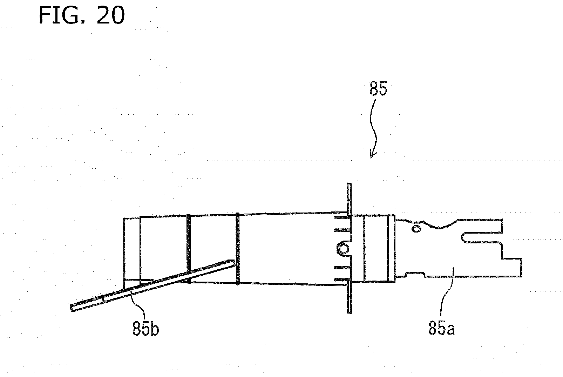

Fig. 19 and Fig. 20 are views showing the fifth embodiment of the optical unit according to the present invention.

An optical unit 1D according to this embodiment has a structure (handpiece type) particularly suitable for being used for medical purposes, such as for examining the inside of the oral cavity by inserting the optical unit, for example.

The optical unit 1D is attached to the distal end side of a cylindrical board case (casing) 80, which is held by the hand. The optical unit 1D includes a cylindrical camera unit 81 and a light guide cylinder 82, the second polarizing element 16 and the second wavelength plate 17 being attached to the distal end of the camera unit 81 in an overlapping manner, the light guide cylinder 82 being disposed to cover the camera unit 81 along the axial direction. A disposable cover 85 (see Fig. 20) can be attached to and detached from the outer peripheral surface of the light guide cylinder 82, the disposable cover 85 being formed into a tubular shape and covering the entire light guide cylinder 82. The disposable cover 85 can be replaced for each patient.

The camera unit 81 is disposed inward of the light guide cylinder 82 in the radial direction, and a locking member 81a is provided to the proximal end side of the camera unit 81. The locking member 81a causes a locking portion 85a, which is integrally formed with the disposable cover 85, to be attached to and detached from the light guide cylinder 82. The disposable cover 85 in this embodiment is configured such that the disposable cover 85 can be attached to and detached from the light guide cylinder 82 in the axial direction. However, a method for attaching and detaching the disposable cover 85 to and from the light guide cylinder 82 is not limited.

In the same manner as the configuration shown in Fig. 3 to Fig. 5, the ring-shaped LED board 5A (LED light source) is provided to the distal end portion of the board case 80, and the lighting-use phase difference ring 10 is disposed in front of the ring-shaped LED board 5A. A ring-shaped first polarizing element and a first wavelength plate are attached to the lighting-use phase difference ring 10 in an overlapping manner. In this case, the first wavelength plate is fixed to a rotary ring 87. By gripping the rotary ring 87 and performing revolving operation, the first wavelength plate can be revolved through 360°.

The above-mentioned optical unit 1D is used to observe the inside of the oral cavity of the patient, and enables observation of the pharynx in the oral cavity. Specifically, when the casing 80 is gripped and the light guide cylinder 82 on which the disposable cover 85 is mounted is inserted into the oral cavity, light from the LED light source is converted to a polarization state and can be emitted from the distal end surface of the light guide cylinder 82, thus irradiating the pharynx. In the same manner as the above-mentioned embodiment, the state of the pharynx can be observed with no glare. Accordingly, for example, in a case where a patient is suffering from influenza (rash occurs in a region of the pharynx), it is possible to diagnose the patient correctly.

As described above, the disposable cover 85 is attached to and detached from the light guide cylinder 82 for each patient. It is preferable that a pressing piece 85b that presses the tongue part is formed at the distal end of the disposable cover 85. By forming such a pressing piece 85b, the tongue can be pressed at the time of performing an examination, thus allowing an affected part to be easily observed and preventing a portion of the light guide cylinder from coming into contact with the oral cavity.

The embodiments of the optical unit according to the present invention have been described heretofore. However, the present invention is not limited to the above-mentioned embodiments, and various modifications are conceivable.

It is sufficient that the casing of the above-mentioned optical unit be configured to allow various optical elements to be disposed in the casing. The shape of the casing is not particularly limited. The light source may be suitably modified, such as the light source being formed of semiconductor laser light emitting elements. Illuminance and a light field diameter brought about by the light source may be suitably modified corresponding to the number and arrangement mode of light emitting elements, refractive index and arrangement number of lenses and the like.

The optical unit according to the present invention can be incorporated in an inspection device for various industrial products. In addition to the above, the optical unit according to the present invention is also applicable in the medical field and the like. For example, the optical unit may be installed in an observation device that obtains intra-operative images, such as an image of the human body, or may be used as a lighting device which is used during operation. The optical unit according to the present invention allows an object to be visually recognized or to be recorded with no glare.

The above-mentioned optical unit is characterized in the layout where the ring-shaped light source is provided, the image pickup element (camera), which receives reflection light from an object, is disposed inward of the ring-shaped light source, the polarizing element 11 is provided to the ring-shaped light source, and the polarizing element 16 is provided to the image pickup element (camera). Therefore, each of the optical units 1, 1A to 1D having the above-mentioned layout may have a configuration where a wavelength plate is not provided to a polarizing element. Further, in a case where a wavelength plate is disposed to overlap with a polarizing element, provided that an inspection object is specified, each wavelength plate may be fixed with a predetermined positional relationship, or may be configured such that the wavelength plate can be attached and detached to change the position of revolution.

### EXPLANATIONS OF LETTERS OR NUMERALS

- 1, 1A, 1B, 1C, 1D: OPTICAL UNIT
- 2, 52, 80: CASING
- 5: LIGHT SOURCE
- 11: FIRST POLARIZING ELEMENT
- 12: FIRST WAVELENGTH PLATE
- 16: SECOND POLARIZING ELEMENT
- 17: SECOND WAVELENGTH PLATE
- 20: CAMERA

## Claims

1. An optical unit that irradiates an object with light and receives reflection light from the object to allow a surface state of the object to be visually recognized, the optical unit comprising:
a light source configured to irradiate the object with light;
a first polarizing element configured to convert emitted light emitted from the light source to linearly polarized light;
a second polarizing element configured to cause the reflection light from the object to transmit through the second polarizing element, a polarization axis of the second polarizing element being 90° orthogonal to a polarization axis of the first polarizing element;
a first wavelength plate disposed to overlap with the first polarizing element and configured to convert the emitted light from the light source to circularly polarized light or elliptically polarized light; and
a second wavelength plate disposed to overlap with the second polarizing element, a phase difference of the second wavelength plate being equal to a phase difference of the first wavelength plate, wherein
the first wavelength plate is revolvable with respect to the first polarizing element disposed to overlap with the first wavelength plate and/or the second wavelength plate is revolvable with respect to the second polarizing element disposed to overlap with the second wavelength plate.

2. The optical unit according to claim 1, wherein
the light source, the first polarizing element, the second polarizing element, the first wavelength plate, and the second wavelength plate are installed in a casing having a cylindrical shape,
the light source is formed into a ring shape, and
the second polarizing element and the second wavelength plate are disposed inward of the light source in a radial direction, the light source having the ring shape.

3. The optical unit according to claim 2, wherein
the light source having the ring shape is formed of a plurality of LEDs disposed at fixed intervals along a circumferential direction, and
each of the plurality of LEDs is provided with a lens that is capable of irradiating the object with spot light, parallel light, or diffused light which is emitted light.

4. The optical unit according to claim 2 or 3, wherein the casing having the cylindrical shape allows hand-held operation, or is supportable by a stand or supportable from a ceiling.

5. The optical unit according to any one of claims 2 to 4, wherein
a camera is installed in the casing, the camera being configured to receive reflection light which is reflected from the object and which transmits through the second polarizing element and the second wavelength plate, and configured to send an object image signal to an external device.

6. The optical unit according to any one of claims 2 to 5, wherein the first wavelength plate and/or the second wavelength plate are/is revolved and controlled by a drive motor installed in the casing.

7. The optical unit according to any one of claims 1 to 6, wherein the first wavelength plate and/or the second wavelength plate are/is operatable at each 1/16 revolution.

8. The optical unit according to claim 1, wherein
the light source, the first polarizing element, the first wavelength plate, the second polarizing element, and the second wavelength plate are incorporated in a mounting member that is mountable on a head of a human body,
the second polarizing element and the second wavelength plate are supported by a support member so as to be disposed in front of each of a left eye and a right eye of the human body, and
the second wavelength plate disposed in front of the left eye and the second wavelength plate disposed in front of the right eye are synchronously revolvable by a synchronization mechanism provided to the support member.

9. The optical unit according to claim 1, wherein
the light source is formed into a ring shape, and is provided to a casing having a cylindrical shape and allowing hand-held operation,
a light guide cylinder is disposed on the casing, the light guide cylinder guiding light which is emitted from the light source having the ring shape and which passes through the first polarizing element and the first wavelength plate, and
a camera is installed inward of the light guide cylinder in a radial direction, the camera being configured to receive reflection light which is reflected from the object and which transmits through the second polarizing element and the second wavelength plate, and configured to send an object image signal to an external device.

10. The optical unit according to claim 9, wherein
the casing is of a handpiece type that is used for a medical purpose, and
a disposable cover is attachable to and detachable from an outer surface of the light guide cylinder.

11. The optical unit according to claim 10, wherein
a pressing piece that presses a tongue part is formed on the disposable cover.
